# EUROPÄISCHE PATENTSCHRIFT

(11) **EP 1 361 869 B1**
(45) Veröffentlichungstag und Bekanntmachung des Hinweises auf die Patenterteilung: **31.08.2011**
(21) Anmeldenummer: 02716736.0
(22) Anmeldetag: 07.02.2002
(51) Int. Cl.: A61K 9/70

(54) **TESTOSTERONHALTIGES TRANSDERMALES THERAPEUTISCHES SYSTEM UND VERFAHREN ZU SEINER HERSTELLUNG**
TRANSDERMAL THERAPEUTIC SYSTEM CONTAINING TESTOSTERONE AND METHOD FOR THE PRODUCTION THEREOF
SYSTEME THERAPEUTIQUE TRANSDERMIQUE CONTENANT DE LA TESTOSTERONE ET SON PROCEDE DE PRODUCTION

(30) Priorität: 19.02.2001 DE 10107663
(43) Veröffentlichungstag der Anmeldung: 19.11.2003
(73) Patentinhaber: LTS LOHMANN Therapie-Systeme AG, 56626 Andernach (DE)
(72) Erfinder: THEOBALD, Frank, 53498 Bad Breisig (DE)
(74) Vertreter: Flaccus, Rolf-Dieter
(86) Internationale Anmeldenummer: PCT/EP2002/001258
(87) Internationale Veröffentlichungsnummer: WO 2002/066018

(56) Entgegenhaltungen:
- WO-A-01/76608
- WO-A-92/10231
- WO-A-96/30000
- WO-A-98/30203
- WO-A-98/32465
- WO-A-99/32153
- DE-A- 3 836 862
- US-A- 5 744 162
- DATABASE WPI Section Ch, Week 200264 Derwent Publications Ltd., London, GB; Class A96, AN 2002-596119 XP002219182 & KR 2002 008 730 A (IL YANG PHARM CO LTD) , 31. Januar 2002 (2002-01-31)

## Beschreibung

Die Erfindung betrifft transdermale therapeutische Systeme (TTS) zur Verabreichung von Sexualhormonen, welche Testosteron und eine Mischung von hautpenetrationsverbessernden Substanzen enthalten. Die Erfindung betrifft ferner Verfahren für die Herstellung solcher TTS.

Testosteron gehört zur Gruppe der Sexualhormone; es ist das stärkste natürliche Androgen. Die tägliche Testosteronproduktion beträgt beim Mann etwa 7 mg (entsprechend 24 µmol) und bei Frauen etwa 10 % dieser Menge. Im Blut ist Testosteron zu 98 % an Transportproteine gebunden. Die Testosteron-Serumkonzentrationen betragen bei Männern 3 bis 10 µg/l, entsprechend 10 bis 35 nmol/l. Sinkt die Serumkonzentration des Testosterons bei Männern unter einen Wert von 10 nmol/l, so spricht man vom Krankheitsbild des Hypogonadismus, welches vor allem durch eine unvollkommene oder fehlende Ausbildung oder sekundäre Rückbildung der primären oder sekundären Geschlechtsmerkmale gekennzeichnet ist. Die Therapie des testosteronmangelbedingten Hypogonadismus besteht in der Substitution von Testosteron.

Aufgrund seiner kurzen Plasmahalbwertszeit (ca. 80 min) und der intensiven first-pass-Metabolisierung ist eine orale Applikation von Testosteron nicht möglich. In der Regel wird es in Form einer geeigneten Esterverbindung durch intramuskuläre Injektion verabreicht.

Andererseits scheint Testosteron aufgrund seiner physikochemischen Eigenschaften zur transdermalen Applikation geeignet zu sein. Allerdings ist hierbei zu beachten, daß die Therapie möglichst unauffällig und diskret erfolgen kann, da der Hypogonadismus eine für den Betroffenen sehr belastende Krankheit darstellt und zur sozialen Ausgrenzung des Betroffenen oder zum Rückzug aus dem sozialen Umfeld führen kann. Dies ist auch bei der Gestaltung eines transdermalen therapeutischen Systems zu berücksichtigen, um die Compliance und damit den Therapie-Erfolg sicherzustellen.

Beispielsweise sind transdermale therapeutische Systeme bekannt, die zur Applikation auf das Skrotum bestimmt sind. Hierbei ist häufig eine Vorbehandlung des Skrotums durch Entfernung der Behaarung erforderlich, wodurch die Benutzerfreundlichkeit und Akzeptanz solcher Systeme beeinträchtigt wird.

Alternativ hierzu existieren transdermale therapeutische Systeme, die als Reservoirsysteme konzipiert sind. Bei derartigen Systemen liegt Testosteron in einem Lösungsmittel, beispielsweise einem Alkohol, gelöst vor. Die Abgabe des Testosterons an die Haut wird mittels einer Kontrollmembran gesteuert. Solche membrangesteuerten Systeme haben den Vorteil, daß sie wie andere aus dem Stand der Technik bekannten TTS auf die Haut appliziert werden können. Sie sind allerdings mit dem Nachteil behaftet, daß es im Falle einer Schädigung der Membran zu einem sogenannten "Dose dumping" kommen kann, d. h. der Inhalt des Wirkstoffreservoirs wird innerhalb kurzer Zeit durch die geschädigte Membran hindurch an die Haut abgegeben, wodurch es zu einer vorübergehenden Überdosierung kommen kann. Darüber hinaus wirken die für das Wirkstoffreservoir üblicherweise verwendeten Lösungsmittel, wie z. B. Alkohole, in den dort verwendeten hohen Konzentrationen häufig hautreizend und verursachen Rötungen und Juckreiz am Applikationsort.

Aus der WO 98/32465 ist ein transdermales therapeutisches System zur Verabreichung von Testosteron oder einem Ester des Testosterons bekannt, das eine Polymermatrix aufweist, die neben dem zu verabreichenden Sexualhormon eine die Penetration von Testosteron durch die Haut fördernde Mischung aus (A) Sorbitanmonolaurat oder Sorbitanmonooleat mit (B) Diethylenglykolmonoethylether oder Diethylenglykolmonomethylether enthält.

Aufgabe der vorliegenden Erfindung war es deshalb, ein transdermales therapeutisches System bereitzustellen, welches die kontinuierliche Abgabe von Testosteron an die Haut ermöglicht, und das nicht mit den vorstehend beschriebenen Nachteilen behaftet ist.

Diese Aufgabe wird durch ein transdermales therapeutisches System (TTS) mit den im Oberbegriff des Anspruchs 1 genannten Merkmalen gelöst, dessen haftklebende Polymermatrix Testosteron und zusätzlich eine Mischung aus mindestens zwei die Hautpenetration verbessernden Substanzen enthält, nämlich mindestens einen penetrationsfördernden Stoff aus der Fettalkoholester und Fettsäureester umfassenden Gruppe und mindestens einen leicht flüchtigen penetrationsfördernden Stoff aus der Isopropylidenglycerol, DEET (= N,N-Diethyl-m-Toluolamid), Solketal, Ethanol, 1,2-Propandiol, kurzkettige Alkohole, Menthol, ätherische Öle und Bestandteile ätherischer Öle umfassenden Gruppe. Gemäß einer bevorzugten Ausführungsform sind sowohl das Hormon als auch die penetrationsfördernden Zusatzstoffe in der haftklebenden Polymermatrix homogen verteilt.

Im Rahmen der der Erfindung zugrunde liegenden Untersuchungen hat sich gezeigt, daß bestimmte Mischungen von Permeationsenhancern (= hautpenetrationsfördernde Substanzen) eine optimale penetrationsfördernde Wirkung für Testosteron bewirken. Dabei handelt es sich um Mischungen aus mindestens einem Fettalkoholester und/oder Fettsäureester, und einer oder mehreren leicht flüchtigen Substanz(en). Bei den leicht flüchtigen Enhancersubstanzen handelt es sich um Isopropylidenglycerol, DEET (= N,N-Diethyl-m-Toluolamid), Solketal, Ethanol, 1,2-Propandiol und andere kurzkettige Alkohole (d. h. Alkohole mit bis zu 6 C-Atomen), sowie Menthol und andere ätherische Öle und Bestandteile ätherischer Öle.

Als Fettalkoholester wird vorzugsweise Ethyloleat verwendet, oder ein Fettalkoholester, der aus der Ethyllaurat, Ethylpalmitat, Ethyllactat, Propyllactat, Propylpalmitat, Propyllaurat, Propyloleat usw. umfassenden Reihe von Verbindungen ausgewählt ist. Als Fettsäureester werden vorzugsweise solche verwendet, die aus der Ölsäureethylester, Ölsäuremethylester, Laurinsäuremethylester, Laurinsäureethylester, Adipinsäuremethylester, Adipinsäureethylester usw. enthaltenden Reihe von Verbindungen ausgewählt sind.

Als besonders geeignet haben sich penetrationsfördernde Mischungen der genannten Art erwiesen, bei denen der/die Stoff(e) aus der Fettalkoholester und Fettsäureester umfassenden Gruppe und der/die leicht flüchtigen Stoffe in einem relativen Mengenverhältnis von 1:2 bis 2:1 vorliegen. Der Anteil des/der leicht flüchtigen penetrationsfördernden Stoffe(s) beträgt dabei vorzugsweise 10 bis 20 Gew.-%, besonders bevorzugt 15 bis 20 Gew.-%, jeweils bezogen auf die Wirkstoffmatrix. Der Anteil des/der penetrationsfördernden Stoffe(s) aus der Fettalkoholester und Fettsäureester umfassenden Gruppe beträgt vorzugsweise 5 bis 20 Gew.-%, besonders bevorzugt 6 bis 10 Gew.-%, jeweils bezogen auf die Matrix (d. h. ohne Berücksichtigung des Vlieses, der Rückschicht und der ablösbaren Schutzschicht).

Des weiteren hat sich herausgestellt, daß durch Zusatz von Nicotinsäureamid zu den erfindungsgemäßen TTS eine weitere Steigerung der Hautpermeationsrate bewirkt werden kann. Die Konzentration des Nicotinsäureamids liegt dabei vorzugsweise im Bereich von 2 bis 10 Gew.-%, besonders bevorzugt im Bereich von 3 bis 5 Gew.-%, jeweils bezogen auf die wirkstoffhaltige Matrix.

Gemäß einer besonders bevorzugten Ausführungsform enthalten die erfindungsgemäßen testosteronhaltigen TTS mindestens einen penetrationsfördernden Stoff aus der Fettalkoholester und Fettsäureester umfassenden Gruppe in einer Gesamtkonzentration von 5 bis 20 Gew.-%, bevorzugt 6 bis 10 Gew.-%, sowie mindestens einen aus der Isopropoylidenglycerol, DEET und kurzkettige Alkohole umfassenden Gruppe ausgewählten Stoff in einer Gesamtkonzentration von 10 bis 20 Gew.-%, bevorzugt 15 bis 20 Gew.-%, und zusätzlich Nicotinsäureamid in einer Konzentration von 2 bis 10 Gew.-%, vorzugsweise 3 bis 5 Gew.-%. Die angegebenen Prozentangaben beziehen sich auf die Matrix.

Der Gehalt an Testosteron liegt bei den erfindungsgemäßen Systemen bevorzugt im Bereich von 0,1 bis 10 Gew.-%, besonders bevorzugt im Bereich von 1 bis 5 Gew.-%, jeweils bezogen auf die Matrix. Unter "Testosteron" werden auch Testosteron-Ester verstanden. Als Testosteron-Ester kommen insbesondere Testosteron-Acetat und Testosteronpropionat in Betracht.

Als haftklebende Matrix wird vorzugsweise eine Polymerschicht verwendet, die auf der Basis von haftklebenden Polymeren aus der Gruppe der Polyacrylate hergestellt ist. Darüber hinaus können auch auf der Basis von Haftschmelzklebern hergestellte Beschichtungen als haftklebende Matrix verwendet werden.

Die haftklebende Polymermatrix kann neben dem/den Polymer(en), dem Wirkstoff und den Enhancersubstanzen noch weitere Hilfsstoffe enthalten, die dem Fachmann bekannt sind. Ansonsten besteht die Matrix im wesentlichen aus haftklebenden Polymeren.

Eine weitere vorteilhafte Ausführungsform sieht vor, daß die erfindungsgemäßen testosteronhaltigen TTS ein Antioxidans oder eine Antioxidans-Kombination enthalten, wobei der Anteil dieser Stoffe vorzugsweise 0,1 bis 5 Gew.-%, besonders bevorzugt 0,3 bis 1 Gew.-% beträgt, jeweils bezogen auf die wirkstoffhaltige Matrix. Als Antioxidans für testosteronhaltige TTS eignen sich vorzugsweise Tocopherol und Ascorbylpalmitat.

Auf der hautabgewandten Seite ist die wirkstoffhaltige Polymermatrix mit einer wirkstoffundurchlässigen Rückschicht bedeckt, die mit der Matrix verbunden ist.

Als Materialien für die Rückschicht eignen sich vor allem Polyester, die sich durch besondere Festigkeit auszeichnen, wie z. B. Polyethylenterephthalat und Polybutylenterephthalat, darüber hinaus aber nahezu beliebige andere hautverträgliche Kunststoffe, wie Polyvinylchlorid, Ethylen-Vinylacetat-Copolymere, Polyvinylacetat, Polyethylen, Polypropylen, Polyurethane, Cellulosederivate und viele andere mehr. Im Einzelfall kann die Rückschicht mit einer zusätzlichen Auflage versehen werden, z. B. durch Bedampfung mit Metallen, insbesondere Aluminium.

Für die ablösbare Schutzschicht können grundsätzlich dieselben Materialien verwendet werden wie für die Rückschicht, vorausgesetzt, daß sie durch eine geeignete Oberflächenbehandlung, wie z. B. Silikonisierung, ablösbar ausgerüstet ist. Es können aber auch andere ablösbaren Schutzschichten wie z. B. mit Polytetrafluorethylen behandeltes Papier oder Cellophan^{®} (Cellulosehydrat) verwendet werden.

Bei der Herstellung von TTS, welche eine wirkstoffhaltige Matrixschicht aufweisen, wird nach dem Stand der Technik üblicherweise so verfahren, daß eine Lösung oder Suspension des Wirkstoffs in einem klebenden oder nicht klebenden Polymer hergestellt wird. Diese Lösung oder Suspension wird mittels eines geeigneten Auftragswerks auf ein Trägermaterial beschichtet und anschließend wird das vorhandene Lösungsmittel durch Trocknung entfernt.

Wenn die herzustellenden Matrix-Systeme, wie im vorliegenden Fall, einen leicht flüchtigen Bestandteil enthalten, ist die beschriebene Vorgehensweise nicht möglich, da es ansonsten zu einem Verdunsten der leicht flüchtigen Komponente kommen würde. Bei der Herstellung der Polymermatrix aus der Schmelze (hot-melt-Verfahren) treten die gleichen Probleme auf.

Erfindungsgemäß wird dieses Problem dadurch gelöst, daß die flüssige Enhancer-Mischung, welche wahlweise zusätzlich den Wirkstoff Testosteron enthalten kann, in definierter Menge auf ein Vlies, Gewebe (z. B. ein textiles Gewebe) oder auf eine Trägerfolie aufgetragen wird. Dieses Vlies, Gewebe oder diese Trägerfolie wird nicht der Trocknung ausgesetzt. Vielmehr wird das so vorbehandelte Vlies oder Gewebe, bzw. die so vorbehandelte Trägerfolie, auf eine bereits vorher hergestellte und getrocknete Polymer-Matrixschicht kaschiert. Das Vlies oder Gewebe ist dann mit der Matrixschicht verbunden und vorzugsweise in diese eingebettet, d. h. es ist Bestandteil der Matrix geworden. Während der nachfolgenden Lagerung kommt es dabei zur Diffusion und damit zur gleichmäßigen, homogenen Verteilung des Wirkstoffs und der Enhancersubstanzen in der Polymermatrix.

Die Mischung der penetrationsfördernden Stoffe, auch als Enhancer-Lösung bezeichnet, kann zwecks Einstellung einer für die Durchführung des vorstehend beschriebenen erfindungsgemäßen Verfahrens geeigneten Viskosität mit Verdickungsmittel oder Gelbildnern versetzt werden. Hierfür eignen sich vorzugsweise Substanzen aus der Polyacrylate, Polyethylenglykol, Polyvinylpyrrolidon, Polyvinylalkohol, Cellulose und Cellulosederivate umfassenden Gruppe.

Eine bevorzugte Ausführungsform des erfindungsgemäßen Herstellungsverfahrens sieht deshalb vor, daß die Herstellung der erfindungsgemäßen Testosteron enthaltenden TTS in der Weise erfolgt, daß zunächst durch Beschichten einer Lösung eines haftklebenden Polymers oder Polymergemisches auf eine folienförmige Unterlage und nachfolgendes Trocknen eine Polymermatrix hergestellt wird. Außerdem wird eine Mischung aus mindestens einem penetrationsfördernden Stoff aus der Fettalkoholester und Fettsäureester umfassenden Gruppe und mindestens einem leicht flüchtigen penetrationsfördernden Stoff zubereitet. Anschließend wird Testosteron zu der vorgenannten Mischung hinzugefügt, wobei Testosteron in der Mischung gelöst wird. Die Zugabe von Testosteron kann entfallen, wenn das Hormon bereits zu der Lösung des haftklebenden Matrixpolymers hinzugegeben wurde.

Die Viskosität dieser flüssigen Enhancer-Mischung kann optional auf die oben beschriebene Weise eingestellt werden.

Anschließend wird die penetrationsfördernde Stoffe (und gegebenenfalls Testosteron) enthaltende Mischung auf ein Vlies oder Gewebe oder eine Trägerfolie aufgetragen. Dieses mit Enhancer-Mischung und gegebenenfalls Testosteron imprägnierte Vlies, Gewebe oder diese Trägerfolie wird auf die getrocknete Polymermatrix kaschiert, so daß es/sie sich mit dieser verbindet oder in diese eingebettet wird. In der Regel befindet sich das Vlies zwischen zwei Polymerschichten eingebettet ("Sandwich").

Testosteron kann bei den vorstehend beschriebenen Herstellungsverfahren auch in Form seiner Ester verwendet werden. Als Testosteron-Ester kommen insbesondere Testosteron-Acetat und Testosteronpropionat in Betracht.

Als Trägerfolie kann die genannte Rückschicht dienen, oder ein für die Rückschicht geeignetes Folienmaterial, wie oben angegeben.

Das Vlies oder Gewebe ist vorzugsweise aus Viskose, Polyester, Polypropylen, Polyethylen, Polyamid, Cellulose, oder aus Kombinationen dieser Materialien hergestellt.

Die Erfindung wird durch die nachfolgenden Beispiele erläutert, ohne sie in irgendeiner Weise einzuschränken:

### Beispiel 1:

| Acrylatmatrix: | |
|---|---|
| | |
| 1. Testosteron | 2,00 % |
| 2. Durotak⁽¹⁾ | 90,70 % |
| 3. Al-Acetylacetonat | 0,80 % |
| 4. Nicotinsäureamid | 5,00 % |
| 5. Tocopherol | 0,75 % |
| 6. Ascorbylpalmitat | 0,75 % |

| Angedickte Enhancerlösung | |
|---|---|
| | |
| 1. Ethyloleat | 21,70 % |
| 2. Solketal | 43,40 % |
| 3. Plastoid B⁽²⁾ | 27,90 % |
| 4. Testosteron | 7,00 % |

Die Acrylatmatrix besitzt ein Flächengewicht von 120 g/m² Die angedickte Enhancerlösung besitzt ein Flächengewicht von 60 g/m²
⁽¹⁾Polyacrylat-Haftkleber (Fa. National Starch)
⁽²⁾Copolymerisat auf der Basis von Methacrylsäure und Methacrylsäuremethylestern (Hersteller: Röhm GmbH)

### Beispiel 2:

Als besonders geeignet hat sich ferner eine Formulierung mit der folgenden Zusammensetzung der Matrixschicht erwiesen:

| |
|---|
| Testosteron..... 3,5 Gew.-% |
| Nicotinsäureamid..... 3,5 Gew.-% |
| Polyacrylat..... 63,0 Gew.-% |
| Ethyloleat..... 10,0 Gew.-% |
| Isopropylidenglycerol..... 20,0 Gew.-% |

(Die Prozentangaben beziehen sich auf die haftklebende Polymermatrix).

Die erfindungsgemäßen Testosteron enthaltenden TTS können in vorteilhafter Weise zur Substitutions-Behandlung des männlichen Hypogonadismus eingesetzt werden.

Darüber hinaus eignen sie sich zur Behandlung anderer testosteronmangelbedingter Krankheitsbilder und Symptome, z. B. zur Behandlung männlicher klimakterischer Symptome ("hormone replacement therapy / HRT" für Männer), Behandlung der männlichen Sterilität oder der androgenmangelbedingten Osteoporose.

Unter Ausnutzung der durch Testosteron vermittelten anabolen Effekte lassen sich die erfindungsgemäßen TTS auch zur unterstützenden Behandlung von HIV-Patienten (AIDS) oder von Tumorpatienten einsetzen, darüber hinaus bei anderen chronisch konsumierenden Erkrankungen oder Krankheitszuständen mit einer katabolen Stoffwechsellage. Ein weiteres bevorzugtes Indikationsgebiet der erfindungsgemäßen testosteronhaltigen TTS betrifft die Behandlung des prämenstruellen Syndroms (PMS) der Frau.

## Patentansprüche

1. Transdermales therapeutisches System zur Verabreichung von Sexualhormonen, welches eine wirkstoffundurchlässige Rückschicht, eine damit verbundene haftklebende PolymerMatrix, die ein Sexualhormon sowie hautpenetrationsfördernde Stoffe enthält, und eine vor der Applikation ablösbare Schutzschicht aufweist, **dadurch gekennzeichnet, daß** die Polymermatrix
- das Sexualhormon Testosteron sowie eine Mischung aus
- mindestens einem penetrationsfördernden Stoff aus der Fettalkoholester und Fettsäureester umfassenden Gruppe, und
- mindestens einem leicht flüchtigen penetrationsfördernden Stoff aus der Isopropylidenglycerol, DEET (= N,N-Diethyl-m-Toluolamid), Solketal, Ethanol, 1,2-Propandiol, kurzkettige Alkohole, Menthol, ätherische Öle und Bestandteile ätherischer Öle umfassenden Gruppe enthält.

2. Transdermales therapeutisches System nach Anspruch 1, **dadurch gekennzeichnet, daß** der/die Stoff(e) aus der Fettalkoholester und Fettsäureester umfassenden Gruppe einerseits und der/die Stoff(e) aus der Gruppe der leicht flüchtigen Stoffe andererseits in der genannten Mischung in einem relativen Mengenverhältnis von 1:2 bis 2:1 vorliegen.

3. Transdermales therapeutisches System nach Anspruch 1 oder 2, **dadurch gekennzeichnet, daß** der Anteil des/der leicht flüchtigen penetrationsfördernden Stoffe(s) 10 bis 20 Gew.-% beträgt, vorzugsweise 15 bis 20 Gew.-%, jeweils bezogen auf die Matrix.

4. Transdermales therapeutisches System nach einem der Ansprüche 1 bis 3, **dadurch gekennzeichnet, daß** der Anteil des/der penetrationsfördernden Stoffe(s) aus der Fettalkoholester und Fettsäureester umfassenden Gruppe 5 bis 20 Gew.-% beträgt, vorzugsweise 6 bis 10 Gew.-%, jeweils bezogen auf die Matrix.

5. Transdermales therapeutisches System nach einem der Ansprüche 1 bis 4, **dadurch gekennzeichnet, daß** es als weitere penetrationsfördernde Komponente Nicotinsäureamid enthält, vorzugsweise in einer Konzentration von 2 bis 10 Gew.-%, besonders bevorzugt in einer Konzentration von 3 bis 5 Gew.-%, jeweils bezogen auf die Matrix.

6. Transdermales therapeutisches System nach Anspruch 1, **dadurch gekennzeichnet, daß** der penetrationsfördernde Stoff aus der Fettalkoholester und Fettsäureester umfassenden Gruppe Ethyloleat ist.

7. Transdermales therapeutisches System nach einem der Ansprüche 1 bis 6, **dadurch gekennzeichnet, daß** die Polymermatrix eine Matrix auf der Basis von Polyacrylaten ist.

8. Transdermales therapeutisches System nach einem der Ansprüche 1 bis 6, **dadurch gekennzeichnet, daß** die Polymermatrix eine Matrix auf der Basis von Haftschmelzklebern ist.

9. Transdermales therapeutisches System nach einem der Ansprüche 1 bis 8, **dadurch gekennzeichnet, daß** die Polymermatrix ein Vlies oder ein Gewebe oder eine Trägerfolie aufweist, welches/welche mit den genannten penetrationsfördernden Stoffen, oder mit den genannten penetrationsfördernden Stoffen und Testosteron imprägniert ist, wobei das Vlies oder Gewebe oder die Trägerfolie mit der Polymermatrix verbunden ist, vorzugsweise in die Polymermatrix eingebettet ist.

10. Transdermales therapeutisches System nach einem der Ansprüche 1 bis 9, **dadurch gekennzeichnet, daß** Testosteron als Ester vorliegt, vorzugsweise als Testosteron-Acetat oder Testosteron-Propionat.

11. Transdermales therapeutisches System nach einem der Ansprüche 1 bis 10, **dadurch gekennzeichnet, daß** der Testosteron-Gehalt 1 bis 10 Gew.-%, vorzugsweise 1 bis 5 Gew.-% beträgt, bezogen auf die Matrix.

12. Transdermales therapeutisches System nach einem der Ansprüche 1 bis 11, **dadurch gekennzeichnet, daß** es ein Antioxidans oder eine Kombination von Antioxidantien, bevorzugt eine Kombination von Tocopherol und Ascorbylpalmitat enthält, wobei der Gehalt des Antioxidans / der Antioxidantien 0,1 bis 5 Gew.-% beträgt, bevorzugt 0,3 bis 1 Gew.-%, jeweils bezogen auf die Matrix.

13. Transdermales therapeutisches System nach einem der Ansprüche 1 bis 12, **dadurch gekennzeichnet, daß** es einen Anteil von Zusatzstoffen aus der Gruppe der Verdickungsmittel und Gelbildner aufweist, vorzugsweise ausgewählt aus der Polyacrylate, Polyethylenglykol, Polyvinylpyrrolidon, Polyvinylalkohol, Cellulose und Cellulosederivate umfassenden Gruppe.

14. Transdermales therapeutisches System nach einem der Ansprüche 1 bis 13, **dadurch gekennzeichnet, daß** der wirkstoff und die penetrationsfördernden Substanzen vollständig gelöst und homogen verteilt im System vorliegen.

15. Verfahren zur Herstellung eines Testosteron und penetrationsfördernde Zusätze enthaltenden transdermalen therapeutischen Systems, **dadurch gekennzeichnet, daß**
- durch Beschichten einer Lösung oder Schmelze eines haftklebenden Polymers oder Polymergemisches auf eine folienförmige Unterlage und nachfolgendes Trocknen eine Polymermatrix hergestellt wird;
- eine Mischung aus mindestens einem penetrationsfördernden Stoff aus der Fettalkoholester und Fettsäureester umfassenden Gruppe und mindestens einem leicht flüchtigen penetrationsfördernden Stoff zubereitet wird;
- Testosteron zu der vorgenannten Mischung hinzugefügt wird, wobei Testosteron in der Mischung gelöst wird;
- die Testosteron und penetrationsfördernde Stoffe enthaltenden Mischung auf ein Vlies oder Gewebe oder eine Trägerfolie aufgetragen wird;
- dieses Vlies, Gewebe oder diese Trägerfolie auf die getrocknete Polymermatrix kaschiert wird.

16. Verfahren zur Herstellung eines Testosteron und penetrationsfördernde Zusätze enthaltenden transdermalen therapeutischen Systems, **dadurch gekennzeichnet, daß**
- durch Beschichten einer Testosteron enthaltenden Lösung oder Schmelze eines haftklebenden Polymers oder Polymergemisches auf eine folienförmige Unterlage und nachfolgendes Trocknen eine Polymermatrix hergestellt wird;
- eine Mischung aus mindestens einem penetrationsfördernden Stoff aus der Fettalkoholester und Fettsäureester umfassenden Gruppe und mindestens einem leicht flüchtigen penetrationsfördernden Stoff zubereitet wird;
- die penetrationsfördernde Stoffe enthaltenden Mischung auf ein Vlies oder Gewebe oder eine Trägerfolie aufgetragen wird;
- dieses Vlies, Gewebe oder diese Trägerfolie auf die getrocknete Polymermatrix kaschiert wird.

17. Verfahren nach Anspruch 15 oder 16, **dadurch gekennzeichnet, daß** zu der penetrationsfördernden flüssigen Mischung mindestens eine Komponente zur Einstellung der Viskosität hinzugefügt wird, wobei diese Komponente vorzugsweise aus der Gruppe der Verdickungsmittel und Gelbildner ausgewählt wird, besonders bevorzugt aus der Polyacrylate, Polyethylenglykol, Polyvinylpyrrolidon, Polyvinylalkohol, Cellulose und Cellulosederivate umfassenden Gruppe.

## Claims

1. Transdermal therapeutic system for administering sex hormones which has an active substance-impermeable backing layer, a pressure-sensitive adhesive polymer matrix connected therewith and containing a sex hormone as well as skin penetration-enhancing substances, and a protective layer detachable prior to application, **characterized in that** said polymer matrix contains
- the sex hormone testosterone as well as a mixture of
- one or more penetration-enhancing substance(s) from the group comprising fatty alcohol esters and fatty acid esters, and
- one or more readily volatile penetration-enhancing substance(s) from the group comprising isopropylidene glycerol, DEET (=N,N-diethyl-m-tolueneamide), solketal, ethanol, 1,2-propanediol, short-chain alcohols, menthol, essential oils and components of essential oils.

2. Transdermal therapeutic system according to claim 1, **characterized in that** the substance(s) from the group comprising fatty alcohol esters and fatty acid esters, on the one hand, and the substance(s) from the group of the readily volatile substances, on the other hand, are present in the said mixture in a relative quantitative ratio of from 1:2 to 2:1.

3. Transdermal therapeutic system according to claim 1 or 2, **characterized in that** the portion of the readily volatile penetration-enhancing substance(s) is 10 to 20%-wt., preferably 15 to 20%-wt., each value relative to the matrix.

4. Transdermal therapeutic system according to any one of claims 1 to 3, **characterized in that** the portion of the penetration-enhancing substance(s) from the group comprising fatty acid esters is 5 to 20%-wt., preferably 6 to 10%-wt., each value relative to the matrix.

5. Transdermal therapeutic system according to any one of claims 1 to 4, **characterized in that** it comprises nicotinamide as further penetration-enhancing component, preferably in a concentration of from 2 to 10%-wt., particularly preferably in a concentration of from 3 to 5%-wt., each value relative to the matrix.

6. Transdermal therapeutic system according to claim 1, **characterized in that** the penetration-enhancing substance from the group comprising fatty alcohol esters and fatty acid esters is ethyl oleate.

7. Transdermal therapeutic system according to any one of claims 1 to 6, **characterized in that** the polymer matrix is a matrix based on polyacrylates.

8. Transdermal therapeutic system according to any one of claims 1 to 6, **characterized in that** the polymer matrix is a matrix based on pressure-sensitive hot-melt adhesives.

9. Transdermal therapeutic system according to any one of claims 1 to 8, **characterized in that** the polymer matrix has a nonwoven fabric or a woven fabric or a carrier film which is/are impregnated with the penetration-enhancing substances mentioned, or with the penetration-enhancing substances mentioned and testosterone, the nonwoven or woven fabric or the carrier film being connected with the polymer matrix, preferably being embedded in the polymer matrix.

10. Transdermal therapeutic system according to any one of claims 1 to 9, **characterized in that** testosterone is present as ester, preferably as testosterone acetate or testosterone propionate.

11. Transdermal therapeutic system according to any one of claims 1 to 10 **characterized in that** the testosterone content amounts to 1 to 10%-wt., preferably 1 to 5%-wt., relative to the matrix.

12. Transdermal therapeutic system according to any one of claims 1 to 11, **characterized in that** it contains an antioxidant or a combination of antioxidants, preferably a combination of tocopherol and ascorbyl palmitate, the content of the antioxidant/antioxidants being 0.1 to 5%-wt., preferably 0.3 to 1%-wt., each value relative to the matrix.

13. Transdermal therapeutic system according to any one of claims 1 to 12, **characterized in that** it has a portion of additives from the group of the thickening agents and gelatinizing agents, preferably selected from the group comprising polyacrylates, polyethylene glycol, polyvinyl pyrrolidone, polyvinyl alcohol, cellulose and cellulose derivatives.

14. Transdermal therapeutic system according to any one of claims 1 to 13, **characterized in that** the active substance and the penetration-enhancing substances are completely dissolved and homogenously distributed in the system.

15. Process for producing a testosterone- and penetration-enhancing additives-containing transdermal therapeutic system, **characterized in that**
- by coating a solution or melt of a pressure-sensitive adhesive polymer or of a polymer mixture onto a film-shaped support and subsequent drying, a polymer matrix is prepared;
- a mixture of at least one penetration-enhancing substance from the group comprising fatty alcohol esters and fatty acid esters and at least one readily volatile penetration-enhancing substance is prepared;
- testosterone is added to the afore-mentioned mixture, dissolving the said testosterone in the mixture;
- the mixture containing testosterone and penetration-enhancing substances is applied to a nonwoven fabric or woven fabric or to a carrier film;
- this nonwoven fabric, woven fabric or carrier film is laminated to the dried polymer matrix.

16. Process for producing a testosterone- and penetration-enhancing additives-containing transdermal therapeutic system, **characterized in that**
- by coating a testosterone-containing solution or melt of a pressure-sensitive adhesive polymer or polymer mixture onto a film-shaped support and subsequent drying, a polymer matrix is prepared;
- a mixture of at least one penetration-enhancing substance from the group comprising fatty alcohol esters and fatty acid esters and at least one readily volatile penetration-enhancing substance is prepared;
- the penetration-enhancing substances-containing mixture is applied to a nonwoven or woven fabric or a carrier film;
- this nonwoven fabric, woven fabric or carrier film is laminated to the dried polymer matrix.

17. Process according to claim 15 or 16, **characterized in that** to the liquid penetration-enhancing mixture is added at least one component for adjusting the viscosity, said component preferably being selected from the group of thickening agents and gelatinizing agents, with particular preference from the group comprising polyacrylates, polyethylene glycol, polyvinyl pyrrolidone, polyvinyl alcohol, cellulose and cellulose derivatives.

## Revendications

1. Système thérapeutique transdermique destiné à l'administration d'hormones sexuelles, qui présente une couche arrière imperméable aux substances actives, une matrice polymère autoadhésive qui est assemblée avec celle-ci, qui contient une hormone sexuelle ainsi que des substances favorisant la pénétration dans la peau, et une couche de protection amovible avant l'application, **caractérisé en ce que** la matrice polymère contient
- l'hormone sexuelle testostérone ainsi qu'un mélange
- d'au moins une substance favorisant la pénétration du groupe comprenant les esters d'alcools gras et les esters d'acides gras et
- d'au moins une substance volatile favorisant la pénétration du groupe comprenant l'isopropylidèneglycérol, le DEET (= N,N-diéthyl-m-toluène-amide), le Solketal, l'éthanol, le 1,2-propanediol, les alcools à courte chaîne, le menthol, les huiles essentielles et les constituants d'huiles essentielles.

2. Système thérapeutique transdermique selon la revendication 1, **caractérisé en ce que** la/les substance(s) du groupe comprenant les esters d'alcools gras et les esters d'acides gras d'une part et la/les substance(s) du groupe des substances volatiles d'autre part se trouvent, dans le mélange mentionné, dans un rapport de quantités relatives de 1:2 à 2:1.

3. Système thérapeutique transdermique selon la revendication 1 ou 2, **caractérisé en ce que** la proportion de la/des substance(s) volatile(s) favorisant la pénétration est de 10 à 20% en poids, de préférence de 15 à 20% en poids, à chaque fois par rapport à la matrice.

4. Système thérapeutique transdermique selon l'une quelconque des revendications 1 à 3, **caractérisé en ce que** la proportion de la/des substance(s) favorisant la pénétration du groupe comprenant les esters d'alcools gras et les esters d'acides gras est de 5 à 20% en poids, de préférence de 6 à 10% en poids, à chaque fois par rapport à la matrice.

5. Système thérapeutique transdermique selon l'une quelconque des revendications 1 à 4, **caractérisé en ce qu'**il contient, comme autre composant favorisant la pénétration, de l'amide de l'acide nicotinique, de préférence en une concentration de 2 à 10% en poids, de manière particulièrement préférée en une concentration de 3 à 5% en poids, à chaque fois par rapport à la matrice.

6. Système thérapeutique transdermique selon la revendication 1, **caractérisé en ce que** la substance favorisant la pénétration du groupe comprenant les esters d'alcools gras et les esters d'acides gras est l'oléate d'éthyle.

7. Système thérapeutique transdermique selon l'une quelconque des revendications 1 à 6, **caractérisé en ce que** la matrice polymère est une matrice à base de polyacrylates.

8. Système thérapeutique transdermique selon l'une quelconque des revendications 1 à 6, **caractérisé en ce que** la matrice polymère est une matrice à base d'autoadhésifs en masse fondue.

9. Système thérapeutique transdermique selon l'une quelconque des revendications 1 à 8, **caractérisé en ce que** la matrice polymère présente un non-tissé ou un tissu ou une feuille support, qui est imprégné(e) avec les substances favorisant la pénétration mentionnées ou avec les substances favorisant la pénétration mentionnées et la testostérone, le non-tissé ou le tissu ou la feuille support étant assemblé(e) avec la matrice polymère, de préférence enrobé(e) dans la matrice polymère.

10. Système thérapeutique transdermique selon l'une quelconque des revendications 1 à 9, **caractérisé en ce que** la testostérone se trouve sous forme d'ester, de préférence sous forme d'acétate de testostérone ou de propionate de testostérone.

11. Système thérapeutique transdermique selon l'une quelconque des revendications 1 à 10, **caractérisé en ce que** la teneur en testostérone est de 1 à 10% en poids, de préférence de 1 à 5% en poids par rapport à la matrice.

12. Système thérapeutique transdermique selon l'une quelconque des revendications 1 à 11, **caractérisé en ce qu'**il contient un antioxydant ou une combinaison d'antioxydants, de préférence une combinaison de tocophérol et de palmitate d'ascorbyle, la teneur en antioxydant/antioxydants étant de 0,1 à 5% en poids, de préférence de 0,3 à 1% en poids, à chaque fois par rapport à la matrice.

13. Système thérapeutique transdermique selon l'une quelconque des revendications 1 à 12, **caractérisé en ce qu'**il présente une proportion d'additifs du groupe des épaississants et des gélifiants, de préférence choisis dans le groupe formé par les polyacrylates, le polyéthylèneglycol, la polyvinylpyrrolidone, le poly(alcool vinylique), la cellulose et les dérivés de cellulose.

14. Système thérapeutique transdermique selon l'une quelconque des revendications 1 à 13, **caractérisé en ce que** la substance active et les substances favorisant la pénétration se trouvent sous forme totalement dissoute et répartie de manière homogène dans le système.

15. Procédé pour la préparation d'un système thérapeutique transdermique contenant de la testostérone et des additifs favorisant la pénétration, **caractérisé en ce que**
- on prépare une matrice polymère par revêtement d'une solution ou d'une masse fondue d'un polymère ou d'un mélange de polymères, autoadhésif, sur un substrat en forme de feuille et séchage consécutif ;
- on prépare un mélange d'au moins une substance favorisant la pénétration du groupe comprenant les esters d'alcools gras et les esters d'acides gras et d'au moins une substance volatile favorisant la pénétration ;
- on ajoute de la testostérone au mélange susmentionné, la testostérone étant dissoute dans le mélange ;
- on applique le mélange contenant la testostérone et les substances favorisant la pénétration sur un non-tissé ou un tissu ou une feuille support ;
- on contre-colle ce non-tissé, ce tissu ou cette feuille support sur la matrice polymère séchée.

16. Procédé pour la préparation d'un système thérapeutique transdermique contenant de la testostérone et des additifs favorisant la pénétration, **caractérisé en ce que**
- on prépare une matrice polymère par revêtement d'une solution ou d'une masse fondue d'un polymère ou d'un mélange de polymères, autoadhésif, contenant de la testostérone sur un substrat en forme de feuille et séchage consécutif ;
- on prépare un mélange d'au moins une substance favorisant la pénétration du groupe comprenant les esters d'alcools gras et les esters d'acides gras et d'au moins une substance volatile favorisant la pénétration ;
- on applique le mélange contenant les substances favorisant la pénétration sur un non-tissé ou un tissu ou une feuille support ;
- on contre-colle ce non-tissé, ce tissu ou cette feuille support sur la matrice polymère séchée.

17. Procédé selon la revendication 15 ou 16, **caractérisé en ce qu'**on ajoute au mélange liquide favorisant la pénétration au moins un composant pour le réglage de la viscosité, ce composant étant de préférence choisi dans le groupe des épaississants et des gélifiants, de manière particulièrement préférée dans le groupe formé par les polyacrylates, le polyéthylèneglycol, la polyvinylpyrrolidone, le poly(alcool vinylique), la cellulose et les dérivés de cellulose.
